(19) **Europäisches Patentamt / European Patent Office / Office européen des brevets**

(11) **EP 1 370 653 B1**

(12) **FASCICULE DE BREVET EUROPEEN**

(45) Date de publication et mention de la délivrance du brevet:
**05.09.2007 Bulletin 2007/36**

(51) Int Cl.:
*C12N 15/09* (2006.01)   *C12Q 1/68* (2006.01)

(21) Numéro de dépôt: **02718262.5**

(22) Date de dépôt: **15.03.2002**

(86) Numéro de dépôt international:
**PCT/FR2002/000937**

(87) Numéro de publication internationale:
**WO 2002/074949 (26.09.2002 Gazette 2002/39)**

(54) **PREPARATION DE CALIBRANTS ET LEUR APPLICATION A LA QUANTIFICATION DE SEQUENCES NUCLEOTIDIQUES D'INTERET**

HERSTELLUNG VON KALIBRATOREN UND IHRE VERWENDUNG ZUR QUANTIFIZIERUNG VON BESONDEREN NUKLEOTIDSEQUENZEN

PREPARATION OF CALIBRANTS AND USE THEREOF IN THE QUANTIFICATION OF NUCLEOTIDE SEQUENCES OF INTEREST

(84) Etats contractants désignés:
**AT BE CH CY DE DK ES FI FR GB GR IE IT LI LU MC NL PT SE TR**

(30) Priorité: **15.03.2001 FR 0103551**

(43) Date de publication de la demande:
**17.12.2003 Bulletin 2003/51**

(73) Titulaire: **Université de la Méditerranée**
**13007 Marseille (FR)**

(72) Inventeurs:
• **GABERT, Jean**
**F-13480 Callas (FR)**
• **BEILLARD, Emmanuel**
**F-13770 Venelles (FR)**

(74) Mandataire: **Peaucelle, Chantal et al**
**Cabinet Armengaud Aîné**
**3, Avenue Bugeaud**
**75116 Paris (FR)**

(56) Documents cités:
• **FUJIMAKI S ET AL: "A quantitative reverse transcriptase polymerase chain reaction method for the detection of leukaemic cells with t(8;21) in peripheral blood." EUROPEAN JOURNAL OF HAEMATOLOGY, vol. 64, no. 4, avril 2000 (2000-04), pages 252-258, XP002205180 ISSN: 0902-4441**
• **BENNETT JULIE M ET AL: "A quantitative PCR method for the assay of HIV-1 provirus load in peripheral blood mononuclear cells." JOURNAL OF VIROLOGICAL METHODS, vol. 83, no. 1-2, décembre 1999 (1999-12), pages 11-20, XP002205181 ISSN: 0166-0934**
• **OLIVERO S ET AL: "Detection of different Ikaros isoforms in human leukaemias using real-time quantitative polymerase chain reaction." BRITISH JOURNAL OF HAEMATOLOGY, vol. 110, no. 4, septembre 2000 (2000-09), pages 826-830, XP002205182 ISSN: 0007-1048**

**EP 1 370 653 B1**

**Description**

La présente invention se rapporte à la préparation de calibrants et à leurs applications à la quantification de séquences nucléotidiques d'intérêt.

La quantification de séquences nucléotidiques d'intérêt peut être réalisée selon plusieurs approches :

- par détermination de la sensibilité minimale d'une expérience (PCR qualitative)
- par utilisation d'un compétiteur (PCR compétitive), ou,
- par PCR quantitative en temps réel.

La PCR qualitative s'attache à la recherche de séquences nucléotidiques. Il s'agit de constater la présence ou l'absence de ces séquences (technique en point final). Par conséquent, aucune quantification précise n'est possible.

La PCR compétitive est une PCR quantitative utilisant un compétiteur chimérique plasmidique. Cette technique suppose que l'efficacité de la PCR est identique pour le compétiteur, et le transcrit recherché, qui sont de tailles différentes. D'autre part, cette technique utilise une PCR nichée qui augmente énormément le risque de contamination.

La PCR quantitative en temps réel vise à quantifier par l'intermédiaire de molécules fluorescentes les acides nucléiques d'un échantillon. Il s'agit d'une technique en temps réel et non en point final. La mesure s'effectue donc durant la phase exponentielle de la PCR, qui permet de supposer un rendement de la PCR de 100%. Dans le domaine de l'oncologie, Marcucci et al. [1] ont été les premiers à décrire cette méthode. Toutefois des problèmes de stabilité ont été constatés au cours de la réalisation de la méthode par les inventeurs. De plus, le rendement d'amplification de la séquence à doser du calibrant n'atteint pas 100% et l'expression moyenne du gène ubiquitaire utilisé pour la normalisation du dosage ne peut être quantifiée de manière correcte. Ces difficultés provoquent alors une non reproductibilité des dosages.

Un autre problème intervenant dans la quantification de gène résulte des variations conséquentes observées pour un même dosage effectué par des laboratoires différents. Les résultats obtenus apparaissent alors dépendants de l'opérateur, du dosage et des conditions dans lesquelles celui-ci opère.

Les travaux des inventeurs les ont conduit à mettre au point une méthode de quantification de séquences nucléotidiques d'intérêt, de manière à s'affranchir de tous les paramètres extérieurs pouvant induire des fluctuations dans les résultats d'un dosage et à obtenir ainsi une reproductibilité parfaite des résultats de ce dosage quel que soit le laboratoire où ce dosage a été réalisé.

L'invention fournit donc un kit de dosage d'une séquence d'ADN d'intérêt à doser, caractérisé en ce qu'il comporte au moins une première gamme de dilution de concentration connues d'une construction (appelée calibrant) comportant une séquence d'ADN correspondant à la séquence d'ADN d'intérêt à doser, insérée dans un vecteur et linéarisée, le milieu de dilution comportant en outre des acides nucléiques ne présentant pas d'homologie avec les séquences d'intérêt desdites constructions, et une seconde gamme de dilutions de concentrations connues d'une construction comportant une séquence d'un gène ou d'un ARN de gène ubiquitaire, insérée dans un vecteur et linéarisée.

Par le terme calibrant, on se réfère à une construction permettant d'effectuer un dosage quantitatif.

La linéarité du calibrant est le paramètre essentiel garantissant la reproductibilité. En effet, la linéarité du calibrant permet d'améliorer considérablement le rendement de la PCR.

Des vecteurs appropriés sont constitués par des vecteurs de clonage.

De préférence, le calibrant est entièrement artificiel.

Le protocole de fabrication d'un calibrant entièrement artificiel est détaillé dans l'exemple 1.

Cette caractéristique permet d'éviter toute interférence dans le dosage qui serait due à la complexité de la matière biologique.

De plus, la séquence d'intérêt est de l'ADN.

En effet, l'utilisation de l'ADN permet une meilleure stabilité dans le temps du calibrant selon l'invention.

Le vecteur utilisé pour la préparation du calibrant selon l'invention pourra être avantageusement un plasmide.

La seconde gamme de dilution va permettre d'effectuer une quantification correcte du gène ou du transcrit de gène ubiquitaire afin de normaliser le premier dosage.

En particulier, la similitude des conditions d'opération des deux dosages permettra la justesse de la normalisation.

Selon un mode de réalisation préféré, le milieu de dilution des kits selon l'invention est une solution tampon.

Le choix de la solution tampon permet de favoriser la stabilité des calibrants lors de leur stockage et pendant les dosages.

La présence d'acides nucléiques dans les milieux des gammes de dilution permet de reproduire artificiellement le milieu biologique de l'échantillon à doser. Il s'agit en effet d'effectuer l'ensemble des dosages dans des conditions similaires afin d'obtenir la meilleure reproductibilité.

De préférence, le milieu de dilution est une solution tampon de pH supérieur ou égal à 7.

Ce pH permet de garantir une stabilité optimum des calibrants.

Selon un mode de réalisation préféré, l'acide nucléique introduit dans les milieux de dilution est de l'ARN d'*E. coli*.

De préférence, le gène ou transcrit de gène ubiquitaire est choisi parmi le groupe composé d'Abelson (ABL), β-glucuronidase (GUS), et β-2microglobuline (B2M).

Ces transcrits de gène ubiquitaire ont été plus particulièrement étudiés par les inventeurs si bien qu'ils sont parfaitement caractérisés (voir le tableau 1 de l'exemple 2). Leur utilisation permet alors d'accroître la reproductibilité d'un dosage à un autre.

Les kits selon l'invention sont particulièrement appropriés pour le dosage de gènes d'intérêt tels que AML1-ETO, E2A-PBX1, BCR-ABL, MLL-AF4, TEL-AML1, SIL-TAL, PML-RARA, CBFb-MYH11. D'où, de manière avantageuse, les kits selon l'invention peuvent comporter des gammes de dilution desdits gènes d'intérêt.

Les séquences et les valeurs de références de ces transcrits de fusion sont décrits dans l'exemple 4.

Selon un mode de réalisation préféré, les gammes de calibrants des kits selon l'invention sont conditionnées sous forme liquide ou lyophilisée.

L'invention couvre également une méthode de préparation d'un kit de dosage d'une séquence nucléotidique d'intérêt, caractérisée en ce qu'elle comporte les étapes suivantes:

- préparation d'une construction comprenant un vecteur, selon la méthode comportant les étapes suivantes :

    (i) insertion d'une séquence d'ADN d'intérêt à doser dans le vecteur, et,
    (ii) linéarisation de ladite construction par une enzyme de restriction sélectionnée de manière telle que ladite enzyme ne reconnaisse qu'un seul site de coupure sur le vecteur, à l'extérieur de l'insert,

- réalisation d'une gamme de dilutions de concentrations connues de ladite construction dans un milieu de pH supérieur ou égal à 7, comportant de l'ARN d'E. *coli*, et,
- réalisation d'une gamme de dilutions de concentrations connues d'une construction comportant une séquence d'un gène ou d'un ARN de gène ubiquitaire, insérée dans un vecteur et linéarisée.

Les calibrants des kits selon l'invention permettent d'effectuer avec précision et reproductibilité, la quantification de toutes sortes d'acides nucléiques dans un échantillon à analyser, avantageusement, d'acides nucléiques associés à des pathologies.

Plus précisément, les calibrants des kits selon l'invention sont particulièrement adaptés à la quantification de gènes, de transcrits de fusion et plus particulièrement à la quantification de transcrits de fusion impliqués dans les leucémies.

L'invention fournit également une méthode de quantification d'un acide nucléique cible dans un échantillon, caractérisée en ce qu'elle comporte les étapes suivantes :

- dosage par PCR quantitative et établissement d'une courbe d'étalonnage à l'aide de la gamme de dilution du calibrant portant la séquence nucléotidique cible,
- dosage par PCR quantitative et établissement d'une courbe d'étalonnage à l'aide de la gamme de dilution d'une construction comportant une séquence d'ADN à doser, insérée dans un vecteur et linéarisée, ladite séquence d'ADN correspondant à la séquence nucléotidique cible,
- dosage par PCR quantitative et établissement d'une courbe d'étalonnage à l'aide de la gamme de dilutions d'une construction portant la séquence du gène ou ARN de gène ubiquitaire, insérée dans un vecteur et linéarisée,
- dosage par PCR quantitative de la séquence cible et du gène ou ARN de gène ubiquitaire dans l'échantillon, à l'aide des courbes d'étalonnage, et,
- calcul du nombre de copies normalisé à l'aide de la formule suivante :

$$\text{Log (NCN)} = \text{Log TF} - \text{Log GU}$$

Où NCN est le nombre de copies normalisé,
TF, le nombre de copies de la séquence cible, et
GU, le nombre de copies du gène ou ARN ubiquitaire,
les dosages étant menés de préférence en double.

Cette méthode est amplement détaillée dans l'exemple 2. Par ailleurs, le dosage effectué dans l'exemple 3 permet de montrer une mise en application de cette méthode.

L'invention propose enfin une méthode de diagnostic d'un patient atteint d'une leucémie impliquant un transcrit de fusion choisi parmi E2A-PBX1, MLL-AF4, TEL-AML1, BCR-ABL(mBCR), BCR-ABL(MBCR), SIL-TAL, PML-RARA, CBFb-MYH11, AML1-ETO comportant les étapes suivantes :

- mise en oeuvre d'un dosage dudit transcrit réalisé selon la méthode de quantification selon la revendication 16, et,
- comparaison du résultat avec une valeur de référence,

et son utilisation pour apprécier l'efficacité d'un traitement anti-leucémique.

Exemple 1 : Préparation d'un calibrant

**I. Brève présentation des différentes étapes du protocole opératoire.**

Un transcrit de fusion (MLL-AF4, par exemple) est amplifié avec les amorces correspondantes [2] à partir de l'ADNc issu d'une lignée cellulaire ou d'un patient. Le produit de PCR est inséré dans un vecteur adapté (plasmide pCR II TOPO par exemple) puis cloné dans une souche bactérienne d'E. *coli* compétente pour la transformation.

Le vecteur plasmidique est alors extrait selon les méthodes classiques [3] et quantifié par spectrophotométrie UV.

Ce vecteur est alors linéarisé. L'enzyme de restriction sélectionnée pour la linéarisation ne doit présenter qu'un seul site de coupure sur le vecteur, à l'extérieur de l'insert.

En tenant compte de la taille du vecteur et de l'insert, la taille de la construction plasmidique est calculée (en paires de base). Son poids moléculaire est estimé pour un poids moyen de nucléotides. Le nombre de copies présent dans un poids donné de plasmide digéré, est alors déterminé.

Une gamme de dilution est ensuite réalisée.

Le standard plasmidique est alors prêt à l'emploi.

**II. Description détaillée du protocole.**

**A. PREPARATION DES SEQUENCES D'ADN A INSERER DANS UN VECTEUR DE CLONAGE**

La préparation des séquences nucléotidiques d'intérêt avant clonage dans un vecteur est effectuée :

- soit à partir d'un ARN transformé en ADNc par une étape de transcription inverse (RT) suivie éventuellement de la synthèse d'un second brin d'ADN,
- soit directement à partir d'un ADN.

Les fragments d'ADN obtenus par l'une ou l'autre des méthodes peuvent ensuite être amplifiés par PCR.

**1. Préparation de séquences d'ADN à partir d'ARN**

Les séquences d'ADN cibles sont d'abord préparées par transcription inverse (RT) d'un ARN total, ribosomal ou messager d'origine humaine, animale ou végétale, d'organismes pro- ou eucaryotes, uni ou pluricellulaires, de virus.

La RT se déroule selon le protocole 1 [2] ou le protocole 2 de RT appliqué aux longs fragments. ➤ Protocole 1 : RT BIOMED-1

Ce protocole est inspiré de la référence [2].

La transcription inverse nécessite des amorces. Elles peuvent être aléatoires (N(6)) ou oligodT(30) comportant éventuellement une séquence additionnelle permettant de réaliser une PCR ancrée (par exemple, SEQ ID N°1 : 5' CGT CGT CGT GAA TTC CTA GAT CTT CTA GAT ATG TT 3'). Les amorces peuvent éventuellement être modifiées à leurs extrémités pour une utilisation ultérieure.

La transcription inverse s'effectue en deux temps :

Dénaturation de l'ARN :

1 $\mu$g d'ARN dans 8,5 $\mu$l d'eau est dénaturé selon le cycle suivant: 10' à 70°C, puis maintien à 20°C.

Synthèse de l'ADNc:

L'ARN dénaturé est mélangé à 10,5 $\mu$l du mélange réactionnel suivant: Tampon 5X(4 $\mu$l), dNTPNa-10 mM (2 $\mu$l), DTT-100mM (2 $\mu$l), amorce de RT 10$\mu$M (1 $\mu$l), RNase-inh 40U/$\mu$l (0,5 $\mu$l), Réverse transcriptase-50U/$\mu$l (2 $\mu$l). La

synthèse de l'ADNc se fait selon le cycle suivant: 10' à 20°C - 45' à 42°C - 3' à 99°C - 4°C infini. Cette synthèse est éventuellement réalisée avec des nucléotides modifiés. ➤ Protocole 2 : RT appliquée aux longs fragments

La transcription inverse nécessite des réactifs qui permettent de former des ADNc de grande taille. Le kit Expand Reverse Transcriptase® de ROCHE est utilisé pour ces expérimentations. Les amorces utilisées sont des amorces aléatoires (N(9)) ou oligodT (30) comportant éventuellement une séquence additionnelle permettant de réaliser une PCR ancrée, telle que SEQ ID N°1 décrite ci-dessus. Les amorces peuvent éventuellement être modifiées à leurs extrémités pour une utilisation ultérieure.

La transcription inverse s'effectue en trois temps :

Dénaturation de l'ARN :

1 $\mu$g d'ARN dans 8,5 $\mu$l d'eau est dénaturé selon le cycle suivant: 10' à 70°C, puis maintien à 20°C.

Synthèse de l'ADNc:

L'ARN dénaturé est mélangé à 10,5 $\mu$l du mélange réactionnel suivant: Tampon 5X(4 $\mu$l), dNTPNa-10 mM (2 $\mu$l), DTT-100mM (2 $\mu$l), amorce de RT 10$\mu$M (1 $\mu$l), RNase-inh 40U/$\mu$l (0.5 $\mu$l), Expand RT-50U/$\mu$l (1 $\mu$l). La synthèse de l'ADNc se fait selon le cycle suivant: 10' à 20°C - 45' à 2h à 42°C - 3' à 99°C - 4°C infini. Cette synthèse peut être réalisée avec des nucléotides modifiés.

Traitement à la RNase H :

2$\mu$l de RNase H (50 U/$\mu$l) sont ajoutés aux 20 $\mu$l du mélange précédent, et incubés 10' à 42°C puis le tube est ramené à 20°C. Le tout est alors dilué au 1/3, volume final 60$\mu$l.

## 2. Séquences d'ADN

Les séquences d'ADN sont obtenues soit directement à partir d'ADN, soit par transcription inverse à partir d'ARN.

Si les séquences cibles sont obtenues directement à partir d'ADN, celui-ci peut être d'origine humaine, animale ou végétale, provenir d'organismes pro- ou eucaryotes, uni ou pluricellulaires, de virus, à partir de séquences introniques, exoniques, codantes ou non codantes.

Si les séquences cibles sont obtenues à partir d'ARN, celui-ci est traité selon le paragraphe précédent afin d'obtenir un premier brin d'ADN complémentaire.

Un second brin d'ADN peut être synthétisé à partir de la séquence obtenue par RT après ajout d'une queue (tailing) en 3', en opérant, par exemple selon le protocole 3 ou ligation avec une séquence prédéfinie contenant éventuellement un site reconnu par une enzyme de restriction à son extrémité. ➤ Protocole 3 : Tailing pour la synthèse d'un second brin

Le tailing est réalisé sur le produit préparé par le protocole 2 et basé sur la référence [3].

Purification de l'ADNc :

La purification est réalisée sur une colonne de Séphadex G50®. L'ADNc est ensuite élué dans 50 $\mu$l d'eau ou de Tris EDTA 10mM/1mM.

Le tailing se réalise comme suit

Dans un volume de 24 $\mu$l : Eau stérile (9,5 $\mu$l) , Tampon (10X) 1,25 $\mu$l, dCTP éventuellement modifié [2mM] 2,5 $\mu$l, MgCl$_2$ [50mM] (0,75$\mu$l), ADNc purifié (10$\mu$l) .

Incuber 2 à 3 minutes à 94 °C. Refroidir 1 mn dans la glace.

Ajouter la TdT, homogénéiser et incuber 15mn à 37°C.

Inactiver la TdT pendant 10 mn à 70°C

Une fois l'ADN obtenu (directement ou indirectement, monobrin ou non), les séquences peuvent éventuellement être amplifiées par PCR ou directement insérées dans un vecteur.

La PCR est réalisée à partir d'un couple d'amorces connues (référence 1) ou d'amorces définies selon l'invention (comportant éventuellement à leur extrémité 5' une séquence reconnue par une enzyme de restriction) permettant d'amplifier la cible selon le protocole 4 de PCR BIOMED-1 ou le protocole 5 de PCR de long fragments. ➤ Protocole 4 : PCR BIOMED-1

Ce protocole de PCR est basé sur la référence [2].

Il permet d'amplifier les séquences d'intérêt ciblées par les amorces (A et B) de la référence [2], et notamment les gènes de fusion de la référence [2] ou des gènes ubiquitaires. Ces amorces peuvent éventuellement être modifiées à leurs extrémités pour une utilisation ultérieure.

Le cycle d'amplification est le suivant: 94°C-3' , 35x(94°C-30" , 65°C-30", 72°C-1'), 16°C-infini. Il est effectué en présence de Taq ADN polymérase®, de nucléotides éventuellement modifiés et de 10 $\mu$l de produit de RT ou de 1 $\mu$l d'ADN. Les fragments obtenus sont de taille courte et peuvent ensuite être clonés dans des vecteurs adaptés. ➤ Protocole 5 : PCR long fragment (classique, ancrée ou bi-ancrée)

Le programme d'amplification et la Taq DNA polymérase® permettent l'obtention de fragments d'au moins 5kb avec des amorces spécifiques. L'amplification est réalisée avec des dNTP éventuellement remplacés par des nucléotides modifiés.

Le cycle d'amplification est le suivant: 94°C-30", 10 x (94°C-10", 68°C-8'), 20 x (94°C-10'', 68°C-8' + 20" par cycle), 68°C-7', 16°C-infini.

Dans un puits de PCR de 50 $\mu$l, le mélange réactionnel est le suivant:

Produit de RT ou de PCR (10$\mu$l), $H_2O$ DEPC (21 $\mu$l), dNTP-10mM (2,5 $\mu$l), Amorce sens ou ancre correspondant à la SEQ ID N°1 ci-dessus de 100$\mu$M (0,5 $\mu$l), Amorce antisens ou ancre de 100$\mu$M (0, 5 $\mu$l), Tampon de PCR (5 $\mu$l), $MgCl_2$ à 3 mM final, Taq ADN polymérase® 3,5U. L'ancre correspond à toute séquence définie, la séquence donnée ici est un exemple comportant des sites de coupure pour des enzymes de restriction.

Cette amplification peut éventuellement être suivie d'une seconde avec des amorces internes pour augmenter la spécificité de l'amplification. Les amorces peuvent éventuellement être modifiées à leurs extrémités pour une utilisation ultérieure.

### 3. Purification des produits de PCR

Aux fins de purification, les produits sont d'abord déposés sur gel pour contrôler la taille et la quantité relative par rapport à un marqueur de poids moléculaire de concentration connue. La quantité du produit est estimée par rapport au marqueur de poids moléculaire. En présence de plusieurs bandes, un isolement de la bande d'intérêt peut être réalisée par découpage sur le gel de la bande recherchée et extraction de l'ADN d'intérêt.

Ces produits sont ensuite éventuellement purifiés par précipitation à l'éthanol, puis repris dans un volume de Tris EDTA 10mM/1mM pour être dosés par spectrophotométrie UV [3] ou par spectrofluorimétrie à l'aide d'un agent fluorescent intercalant.

### B . CLONAGE DES SEQUENCES

Les produits préparés sont ensuite clonés. A cet effet, ils sont insérés dans un vecteur de clonage, puis transférés dans un organisme hôte d'origine humaine, animale, virale ou végétale, d'organismes pro- ou eucaryotes, uni ou pluricellulaires, afin de produire la séquence d'intérêt en grande quantité.

### 1. Production de la séquence dans des organismes procaryotes ou viraux

La (es) séquence (s) d'intérêt est (sont) liguée (s) au vecteur par une ligase ou une topoisomérase au site de clonage. Le vecteur plasmidique (BlueScript® ou équivalent), phagique (Lambda ou équivalent), cosmidique, viral ou encore un vecteur YAC (levures) ou BAC contenant l'insert est amplifié en grande quantité pour être extrait.

La cellule hôte réceptrice est avantageusement une souche bactérienne *Escherichia coli,* rendue compétente pour la transformation par une méthode physique ou chimique. Le clone (vecteur + insert) peut être introduit dans d'autres souches bactériennes ou organismes procaryotes, par exemple des levures ou des cellules d'insecte. L'extraction du vecteur porteur de l'insert est réalisée selon les techniques classiques [3].

### 2. Production de la séquence dans des organismes eucaryotes

La (es) séquence (s) d'intérêt est (sont) insérée (s) dans des vecteurs eucaryotes par une ligase ou une topoisomérase au site de clonage, puis transférée(s) dans des organismes eucaryotes unicellulaires (par exemple, *Saccharomyces sp* ou *Candida sp),* pluricellulaires ou dans des lignées immortalisées humaines, animales ou végétales.

### 3. Contrôle qualitatif de l'insert et séquençage

La sélection de l'organisme porteur du vecteur est réalisée avantageusement selon les méthodes habituelles [3] et la présence de l'insert est vérifiée par PCR en choisissant des amorces non spécifiques dessinées sur le vecteur ou spécifiques de l'insert en opérant en particulier selon le protocole 4 des exemples.

Le séquençage du produit inséré est réalisé pour déterminer la présence d'éventuelles mutations dans le produit cloné et le sens d'insertion du produit. Le séquençage est réalisé selon les technologies classiques à partir d'amorces universelles situées sur le vecteur de clonage (M13, Sp6 ou T7 par exemple) ou à partir des amorces spécifiques à l'insert.

## C. QUANTIFICATION DU GENE D'INTERET

La calibrant peut comporter le gène d'intérêt (insert) sous différentes formes : ADN double ou simple brin, ARN.

### 1. ADN double brin

Le vecteur de clonage est extrait des cellules par les techniques classiques d'extraction (SAMBROOK). Le produit est repris dans une solution de Tris EDTA 10mM/1mM pH=8, quantifié par spectrophotométrie UV [3] ou par spectrofluorimétrie à l'aide d'un agent intercalant.

Puis 20 $\mu$g de ce produit sont digérés dans 20 $\mu$l pendant une heure à 37°C avec 10 U d'une enzyme de restriction appropriée (BamHI, HindIII, ou autre) pour linéariser le vecteur.

Le nombre de copies de la séquence d'intérêt ou son équivalent en unité de masse est ensuite déterminé en tenant compte du poids moléculaire du vecteur, de la taille de l'insert et de la quantité du produit cloné dans un volume déterminé (Formule 1)

$$\text{Formule 1 : } PM_C = (NTV + NTI) * PM_N / N$$

où $PM_C$ est le poids moléculaire (g) d'une copie de vecteur portant 1 insert,
NTV, le nombre de nucléotides du vecteur,
NTI, le nombre de nucléotides de l'insert,
$PM_N$, le poids moléculaire moyen d'un nucléotide, soit 321,44g, et,
$N$, le nombre d'Avogadro, soit $6.02 \times 10^{23}$.

### 2. ADN simple brin

A partir du vecteur linéarisé (voir le paragraphe précédent), porteur des séquences universelles M13F et M13R, une séquence d'ADN simple brin est produite en utilisant une polymérase (type KLENOW ou équivalent) en présence de l'amorce correspondante pour obtenir le brin souhaité, selon les protocoles classiques (SAMBROOK). Le produit est ensuite purifié sur gel puis repris dans une solution de Tris EDTA 10mM/1mM pH=8, quantifié par spectrophotométrie UV [3] ou par spectrofluorimétrie à l'aide d'un agent intercalant.

Le nombre de copies de la séquence d'intérêt ou son équivalent en unité de masse est ensuite déterminé en tenant compte de la taille de l'insert et de la quantité du produit dans un volume déterminé (Formule 1).

### 3. ARN

Pour préparer de l'ARN, on met à profit le fait que le vecteur de clonage (pCR II TOPO ou Blue Script) porteur de la séquence d'intérêt décrit plus haut possède les promoteurs pour les ARN polymérases SP6 ou T7. Ces polymérases sont utilisées pour la production d'ARN simple brin à partir des sites promoteurs sur le vecteur selon les protocoles habituels [3]. Le choix de la polymérase est fonction du sens d'insertion de la séquence d'intérêt.

L'ARN produit est alors purifié selon les techniques classiques d'isolement [3]. Puis il est dosé par spectrophotométrie UV [3] ou par spectrofluorimétrie à l'aide d'un agent intercalant (Hoescht ou Interchim).

Enfin, le nombre de copies de la séquence d'intérêt ou son équivalent en unité de masse est déterminé en tenant compte du poids moléculaire de l'insert et de la quantité du produit cloné dans un volume déterminé (Formule 1).

## D. DILUTION DES PREPARATIONS

Les séquences d'intérêt sont ensuite diluées de manière à connaître leur quantité exacte dans un volume déterminé.

Le produit final peut être un échantillon ou une série de dilutions en cascade de l'échantillon comportant une seule ou plusieurs séquences d'ARN et/ou d'ADN simple ou double brin, véhiculé par le même fragment d'acide nucléique ou par des fragments différents.

La quantité peut être exprimée sous forme d'unité de masse de la séquence de référence ou en nombre absolu ou relatif par rapport à une seconde séquence également présente en quantité connue dans la préparation. Par extension, un mélange d'un grand nombre de séquence de quantité connue dans un même échantillon peut être préparé de la même manière.

Le produit final peut être lyophilisé ou en solution dans un solvant approprié, comportant éventuellement des agents inertes ou actifs.

## 1. Réalisation pratique des dilutions d'ADN

Pour réaliser des dilutions d'ADN, à partir d'une quantité connue d'ADN simple ou double brin, on effectue une dilution en cascade de 10 en 10 ou de demi en demi de cet échantillon. Ces dilutions sont effectuées dans un ARN (dilutions réalisées dans un ARN d'origine humaine, animale ou végétale, d'organismes pro- ou eucaryotes, uni ou pluricellulaires, de virus) ou un ADN en solution dans un tampon TRIS EDTA 10mM/1mM pH=8 en présence d'une ou plusieurs substances inertes (glycérol, Sérum albumine bovine, lait en poudre, Dextran, ARN ribosomal eucaryotique 16 et 23S, ou tout autre produit chimique stabilisant).

## 2. Réalisation pratique des dilutions d'ARN

Pour réaliser des dilutions d'ARN, on dilue une quantité connue d'ARN produit en cascade de 10 en 10 ou de demi en demi. Ces dilutions sont réalisées dans un ARN (d'origine humaine, animale ou végétale, d'organismes pro- ou eucaryotes, uni ou pluricellulaires, de virus) ou un ADN en solution dans un tampon TRIS EDTA 10mM/1mM pH=8 en présence d'une ou plusieurs substances inertes (glycérol, Sérum albumine bovine, lait en poudre, Dextran, ou tout autre produit chimique stabilisant).

Par exemple, la première dilution appelée $10^{-1}$ comportera $2.10^9$ copies du plasmide par microlitre de solution, obtenue à partir d'un volume V de la digestion enzymatique diluée dans un volume final de 500$\mu$l d'eau DEPC. Des dilutions en cascade sont ensuite réalisées dans un tampon Tris EDTA 10mM/1mM pH=8 contenant 20ng/$\mu$l d'ARN 16S&23S d'E. coli pour obtenir des concentrations finales de 200000, 20000, 2000, 200, 20 et 2 copies par microlitre de solution.

On procède ensuite au contrôle des préparations. **E. CONTROLE DES PREPARATIONS**

## 1. Contrôle qualitatif de la présence de l'insert

Les contrôles qualitatifs de la présence de l'insert sont réalisés pour déterminer la présence de l'insert recherché dans le produit cloné. L'aspect des (de la) bande(s) amplifiée(s) sur gel permet d'apprécier la qualité de la préparation. Ce contrôle s'effectue par PCR à partir d'amorces universelles situées sur le vecteur de clonage ou à partir des amorces qui ont servi à produire le fragment de PCR selon le programme d'amplification BIOMED-1. Il s'effectue de manière directe à partir des échantillons d'ADN ou après RT (en particulier selon le protocole 1 ou 2 à partir des échantillons d'ARN.

## 2. Contrôle quantitatif de la présence de l'insert

Les contrôles quantitatifs sont réalisés pour déterminer la quantité de séquence d'intérêt ou de vecteur. Ce contrôle peut s'effectuer par PCR à partir d'amorces universelles situées sur le vecteur de clonage ou à partir des amorces qui ont servi à produire le fragment de PCR selon le programme d'amplification du protocole 6. ➤ Protocole 6 : PCR quantitative de type Taqman®

Le cycle d'amplification est le suivant :

50°C-2' , 94°C-2', 50 x (94°C-30" , 60°C-1'), 16°C-infini.

La composition du mélange réactionnel est la suivante :

- Echantillon d'ADN ou d'ADNc (100 ng d'ADN ou son équivalent)
- Amorces sens et antisens 300 nM final
- Sonde de révélation Taqman® marquée de fluorophores 200 nM final ou son équivalent (agent intercalant fluorescent, SYBR green© par exemple) permettant la révélation du produit amplifié ou mélange réactionnel contenant ces composants,
- Mélange réactionnel contenant le tampon de PCR, la polymérase, et tout autre composant permettant la quantification.

Le contrôle est réalisé de manière directe à partir des échantillons d'ADN ou après RT à partir des échantillons d'ARN (notamment selon le Protocole 1 ou 2). La quantification peut également être réalisée par les techniques actuelles de mesure des séquences nucléotidiques, en solution ou sur supports solides grâce à des substances adaptées à la quantification.

On observera que, hormis les produits obtenus à partir d'une dilution directe du vecteur (ADN double brin) dans la solution tampon, les séquences du vecteur n'interviennent à aucun moment dans le produit final.

## EXEMPLE 2 : METHODE DE QUANTIFICATION

La quantification comporte deux étapes principales :

- la première étape consiste à doser le gène d'intérêt,
- la seconde étape a pour but de normaliser le dosage effectué.

La normalisation s'effectue par le dosage d'un gène dit « ubiquitaire ».

Tous les dosages sont effectués par PCR quantitative.

Pour chaque étape, on établit une courbe d'étalonnage, à l'aide du calibrant selon l'invention, dite « courbe standard plasmidique ». On effectue ensuite le dosage du gène d'intérêt et du gène ubiquitaire, présents dans l'échantillon. Les valeurs du dosage sont ensuite déterminées par interpolation, sur la courbe d'étalonnage.

### A. Quantification du gène ou transcrit de gène ubiquitaire dans l'échantillon

Par «gène ubiquitaire », on entend un gène de référence servant à normaliser les expériences. Sa quantification est précise et reproductible d'expérience à expérience.

Il peut également s'agir du transcrit du gène ubiquitaire. Il existe de nombreux gènes ubiquitaires (β-globine, β-actine, GAPDH ou des transcrits des gènes comme Abelson, β-2 microglobuline ou B2M, β-glucuronidase ou GUS, Porphobilinogène déaminase ou PBGD , Tata Box binding protéine ou TBP, etc ...) dont on connaît approximativement l'expression dans les cellules du corps humain.

A titre d'exemple, les gènes ubiquitaires préférés ainsi que leurs valeurs expérimentales, sont décrits dans le tableau suivant :

**Tableau 1 :**

|  | N | $10^{CN}$ moy. | Plage |
|---|---|---|---|
| **ABL** |  |  |  |
| Tous éch. | 316 | 18071 | 1345-127643 |
| Ech. Leucémique | 190 | 13378 | 1175-151356 |
| Ech. « normaux » | 126 | 19953 | 1380-53703 |
| **B2M** |  |  |  |
| Ech. Leucémique | 190 | 781628 | 9977-5370317 |
| SP&CSSP« normaux» | 100 | 1445440 | 208929-6760829 |
| Moëlle «normale» | 26 | 1071519 | 28641-1927524 |
| **GUS** |  |  |  |
| Ech. Leucémique | 190 | 40926 | 2944-344349 |
| SP& CSSP « normaux » | 52 | 66069 | 7177-195434 |
| Moëlle « normale » | 74 | 21878 | 2630-70794 |
| N: nombre d'échantillons<br>$10^{CN}$ moy.: nombre moyen de copies dans un échantillon pour 100 ng d'ARN total issu de cet échantillon.<br>Plage : valeur supérieure et inférieure de la plage comportant 95% des échantillons.<br>Ech. : échantillon<br>SP : sang périphérique<br>CSSP : cellules souches du sang périphérique |  |  |  |

Le gène ubiquitaire est choisi en fonction de divers paramètres :

- il faut en premier lieu que son expression soit stable dans les conditions expérimentales,
- il faut également que son expression soit corrélée avec celle de la cible au cours du processus de dégradation, c'est-à-dire que son expression reste dans un rapport constant avec celle de la cible.

Le gène ubiquitaire est préparé selon la même méthode que le gène d'intérêt. On obtient ainsi une gamme de dilution de calibrants contenant le gène ubiquitaire.

Le dosage du « gène ubiquitaire » ou de son transcrit va donner une information sur la qualité du dosage effectué. En effet, le dosage s'effectue par PCR quantitative ce qui peut traduire une dégradation de l'ADN ou de l'ARN dosé, en particulier dans l'échantillon qui représente un milieu complexe. Il est supposé que cette dégradation de l'ADN ou de l'ARN dosé va s'accompagner d'une dégradation similaire, dans les mêmes proportions, du « gène ou transcrit ubiquitaire ». Si on connaît la quantité exacte de gène ubiquitaire déterminé par la PCR, on peut en déduire la dégradation dudit gène et d'après le postulat énoncé ci-dessus, celle du gène d'intérêt à doser dans l'échantillon.

Une courbe standard plasmidique est établie sur 3 dilutions logarithmiques dosées en double dans la même expérience. La position du premier et du second point de dilution est déterminée par rapport à l'expression moyenne observée du gène étudié. Le nombre de copies présentes dans l'échantillon considéré est déterminé par interpolation.

Par exemple, l'expression moyenne du gène ubiquitaire ABL est de 20 000 copies pour 0,1 $\mu$g d'équivalent d'ARN. La première dilution de plasmide est calculée pour contenir 100 000 copies dans 5 $\mu$l de solution. La seconde et la troisième contiendront respectivement 10 000 et 1000 copies dans 5$\mu$l de solution. De cette façon, toute dégradation de l'ARN de l'échantillon se traduira par un nombre de copies plus faible et sera encadré par les deuxième et troisième dilutions.

**B. Quantification de la séquence d'intérêt**

Une courbe standard plasmidique est établie sur 4 à 5 dilutions logarithmiques dosées en double dans la même expérience. Le premier point de gamme correspond à 1 000 000 copies du gène et le dernier à 10 copies. Les dilutions intermédiaires sont 100 000, 1000 et (ou) 100 copies. Le nombre de copies présentes dans l'échantillon considéré est déterminé par interpolation.

**C. Expression des résultats**

L'amplification des produits de PCR suit une loi géométrique. La distribution statistique du nombre de copies dans un échantillon pour un dosage répété est logarithmique. Il est donc nécessaire de considérer les valeurs logarithmiques pour effectuer les calculs de normalisation.

Ainsi, pour un échantillon dosé en double pour un gène d'intérêt donné, par exemple, un transcrit de fusion (TF1 et TF2) et son gène ubiquitaire (GU1 et GU2), la valeur obtenue suit l'expression suivante :

> **Formule 2**
>
> ```
> Log (NCN) = (Log TF1 + Log TF2)/2 - (Log GU1 + Log GU2)/2 + CN
> ```

Où NCN est le nombre de copies normalisé,
TF, le nombre de copies de la séquence cible (par exemple, transcrit de fusion),
GU, le nombre de copies du gène ou transcrit de gène ubiquitaire,
CN (facultatif) est le logarithme du nombre médian de copies observé pour le gène considéré (ex : pour ABL, CN = 4). Cela permet d'exprimer le résultat pour 10 000 copies d'ABL.

Par le terme « Log », on se réfère à une logarithme décimal.

Les résultats peuvent ensuite être rapportés à des unités dérivées : au nombre de mole, de grammes ou leurs dérivés.

**EXEMPLE 3 : DOSAGE D'UN TRANSCRIT D'UNE CIBLE RAPPORTE A UN TRANSCRIT DE REFERENCE**

On réalise en parallèle l'amplification d'un transcrit de fusion (MLL-AF4) et d'un gène ubiquitaire (ABL) par PCR quantitative. Les résultats bruts de la machine sont portés dans le tableau suivant.

**Tableau 2:**

| $10^{CN}$ | MLL-AF4 (Ct) | | ABL (Ct) | |
|---|---|---|---|---|
| | Réplicat 1 | Réplicat 2 | Réplicat 1 | Réplicat 2 |
| Patient | 27.9 | 28.1 | 26.98 | 27.02 |
| Plasmide $10^6$ copies | 19.35 | 19.26 | | |
| Plasmide $10^5$ copies | 22.84 | 22.85 | 23.46 | 23.55 |
| Plasmide $10^4$ copies | | | 27.43 | 27.09 |
| Plasmide $10^3$ copies | 30.42 | 30.59 | 30.3 | 30.5 |
| Plasmide $10^2$ copies | 34.74 | 37.79 | | |
| Plasmide $10^1$ copies | 37.17 | 40.18 | | |
| *Droite de régression* | Ct = -3.88 CN + 42.4 | | Ct = -3.43 CN + 40.8 | |
| *Résultats patient* | CN = 3.71, soit 5149 copies | | CN = 4.01, soit 10272 copies | |

Exemple d'amplification en parallèle de 2 transcrits ABL et MLL-AF4 sur des plasmides et un échantillon de patient au diagnostic.

Les valeurs brutes (Ct) des dilutions plasmidiques données par le machine sont utilisées pour construire la droite de régression en portant en abscisse le log du nombre de copies et en ordonnée le Ct de la dilution. Les droites de régression correspondantes sont portées sur le tableau 2. Le nombre de copies correspondant est calculé par interpolation.
En utilisant la formule 2, le résultat obtenu pour le patient est calculé selon :

$$\text{Log(NCN)} = 3.71-4.01 = -0.30$$

d'où
NCN = $10^{(-0.30)}$ = 0.501 copie de MLL-AF4 par copie du transcrit de référence (ABL).

## EXEMPLE 4 : DOSAGE DE TRANSCRITS IMPLIQUES DANS LES LEUCEMIES

### 1. Présentation des transcrits

Les transcrits de fusion résultent de la fusion de deux gènes.
Le tableau ci-dessous fournit les numéros d'accession dans la banque de données Genbank®, des séquences des deux gènes ayant fusionnés pour produire le transcrit décrit.

**Tableau 3 :**

| Transcrit de fusion N° | accession gène 1 | N° accession gène 2 |
|---|---|---|
| AML1-ETO | D43369 | D14289 |
| E2A-PBX1 | M31222 | M86546 |
| BCR-ABL | X02596 | X16416 |
| MLL-AF4 | L04284 | L13773 |
| TEL-AML1 | U11732 | D43369 |
| SIL-TAL | M74558 | S53245 |
| PML-RARA | M73778 | X06538 |
| CBFb-MYH11 | L20298 | D10667 |

**2. Détermination des valeurs de référence**

Les valeurs de niveau d'expression des principaux transcrits de fusion impliqués dans les leucémies n'ont jusqu'à présent jamais été présentées dans leur globalité.

Les tableaux suivants illustrent ces valeurs. Elles sont utilisables en tant que valeur de comparaison suite à un dosage, pouvant être réalisé selon la méthode quantification selon l'invention. Ces dosages peuvent en particulier être destinés au diagnostic (dans le cadre d'une méthode de diagnostic, par exemple) ou pour apprécier l'efficacité d'un traitement concernant tout état pathologique pour lequel il a été mis en évidence au diagnostic une anomalie génique (gène ou transcrit) détectable par la méthode proposée. En particulier, les méthodes de dosages et de diagnostic selon l'invention permettent le suivi de traitements anti-leucémiques.

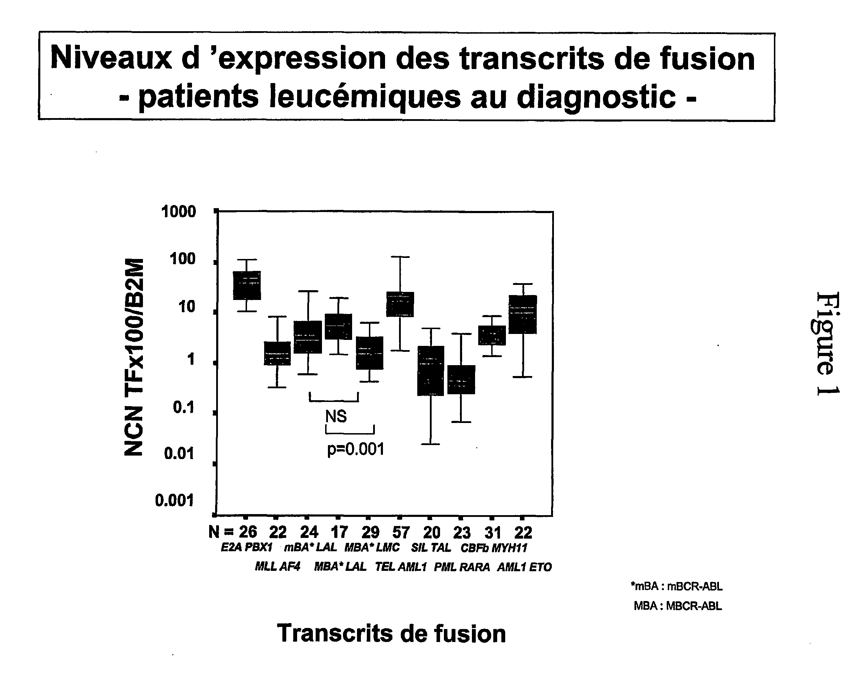

L'ensemble de ces valeurs sont résumées dans la figure 1. Dans cette figure, TG se réfère aux transcrits de fusion, NCN, au nombre de copies normalisé, LAL, à une leucémie aiguë lymphoblastique et LMC, à une leucémie myéloïde chronique.

Dans les tableaux suivants, sauf exception, le nombre de copies normalisé du gène de fusion est donné pour une copie du gène ABL, du gène GUS ou 100 copies de B2M. La valeur médiane est définie pour une plage couvrant 95% des valeurs. Le coefficient de corrélation (Correl. Coef.) est défini avant normalisation par le gène ubiquitaire.

Aucune différence statistiquement significative n'a été constatée entre les résultats obtenus sur les échantillons de sang et ceux de la moelle.

**Tableau 4 : Amplification des gènes ABL, B2M, GUS et E2A-PBX1 dans la lignée cellulaire et chez des patients au diagnostic.**

| | Nombre de Copies normalisé | | |
|---|---|---|---|
| Echantillons | E2A-PBX1/ ABL | E2A-PBX1x100/ B2M | E2A-PBX1/ GUS |
| Lignée cellulaire 697 | 5.0 | 33.9 | 9.0 |
| Patients Moelle (n=14) | 7.6 [2.1-19.5] | 44.6 [10.5-101.0] | 8.9 [1.6-17.8] |
| sang (n=13) | 9.4 [3.8-72.4] | 42.7 [2.2-115.9] | 13.6 [2.5-67.6] |
| Correl. Coef. | 0.83 | 0.62 | 0.66 |

**Tableau 5 : Amplification des gènes ABL, B2M, GUS et MLL-AF4 dans des lignées cellulaires et chez des patients au diagnostic.**

| | Nombre de Copies normalisé | | |
|---|---|---|---|
| Echantillons | MLL-AF4/ ABL | MLL-AF4x100 /B2M | MLL-AF4/GUS |
| Lignées cellulaires | | | |
| RS4 ; 11 | 0.35 | 4.4 | 0.29 |
| MVA-11 | 0.56 | 9.3 | 0.63 |
| ALL-PO | 0.62 | 9.3 | 0.59 |
| Patients Moelle (n=14) | 0.46 [0.09-0.98] | 1.2 [0.07-8.3] | 0.32 [0.04-0.65] |
| sang (n=13) | 0.60 [0.16-0.91] | 1.4 [0.32-2.9] | 0.27 [0.09-0.58] |
| Correl. Coef. | 0.79 | 0.67 | 0.76 |

**Tableau 6 : Amplification des gènes ABL, B2M, GUS et TEL-AML1 dans la lignée cellulaire REH et chez des patients au diagnostic.**

| Echantillons | Nombre de Copies normalisé | | |
| --- | --- | --- | --- |
| | TEL-AML 1/ABL | TEL-AML1×100/ B2M | TEL-AML1/ GUS* |
| Lignée cellulaire REH | 3.2 | 27 | 2.5 |
| Patients Moelle (n=30) | 4.45 [0.46-32.70] | 23 [0.63-132] | 3.19 [0.27-10.33] |
| sang (n=13) | 4.71 [0.20-15.2] | 12 [0.39-99] | 2.63 [0.50-9.00] |
| Correl. Coef. | 0.66 | 0.42 | 0.66 |

* 12 échantillons de sang et 17 échantillons de moelle ont été testés pour GUS.

**Tableau 7 : Amplification des gènes ABL, B2M, GUS et BCR-ABL(mBCR) dans la lignée cellulaire et chez des patients au diagnostic.**

| Echantillons | Nombre de Copies normalisé | |
| --- | --- | --- |
| | BCR-ABL (mBCR)×100/B2M | BCR-ABL (mBCR)/GUS |
| Patients Moelle (n=17) | 2.69 [0.58-26.3] | 1.48 [0.24-16.6] |
| sang (n=13) | 3.16 [0.03-13.8] | 1.62 [0.18-9.33] |

**Tableau 8 : Amplification des gènes ABL, B2M, GUS et BCR-ABL(MBCR) dans la lignée cellulaire et chez des patients au diagnostic.**

| Echantillons | Nombre de Copies normalisé | |
| --- | --- | --- |
| | BCR-ABL(MBCR)x100/ B2M | BCR-ABL(MBCR)/ GUS |
| Lignée cellulaire K562 | 2.24 | 0.66 |
| Patients Moelle (n=15) | 0.95 [0.43-5.3] | 0.12 [0.04-0.87] |
| Sang (n=14) | 2.8 [0.56-6.2] | 0.22 [0.08-0.41] |
| Correl. coef. | 0.63 | 0.76 |
| Patients Moelle et Sang (n=17) | 4.90 [0.14-19.5] | 1.15 [0.03-3.89] |
| Correl. coef. | 0.56 | 0.60 |

**Tableau 9 : Amplification des gènes ABL, B2M, GUS et SIL-TAL dans des lignées cellulaires et chez des patients au diagnostic.**

| Echantillons | Nombre de Copies normalisé | | |
|---|---|---|---|
| | SIL-TAL /ABL | SIL-TAL /100B2M | SIL-TAL /GUS |
| Lignée cellulaires | | | |
| **CEM** | 0.12 | 1.38 | 0.07 |
| ALL1 | 0.11 | 0.79 | 0.09 |
| Molt-15 | 0.10 | 0.27 | 0.06 |
| Molt-16 | 0.08 | 0.45 | 0.11 |
| HSB2 | 0.41 | 3.47 | 0.44 |
| PF382 | 0.07 | 0.47 | 0.11 |
| Patients | | | |
| Moelle | 0.12 | 1.24 | 0.15 |
| (n=10) | [0.02-0.23] | [0.03-4.86] | [0.02-0.44] |
| Sang | 0.09 | 0.86 | 0.12 |
| (n=10) | [0.02-0.21] | [0.05-3.28] | [0.01-0.26] |

**Tableau 10 : Amplification des gènes ABL, B2M, GUS et PML-RARA chez des patients au diagnostic.**

| Echantillons | Nombre de Copies normalisé | | |
|---|---|---|---|
| | PML-RARA / ABL | PML-RARA x100/B2M | PML-RARA / GUS |
| Patients | | | |
| Moelle | 0.30 | 0.72 | 0.08 |
| (n=14) | [0.09-1.82] | [0.19-3.18] | [0.02-0.56] |
| Sang | 0.36 | | 0.05 |
| (n=9) | 0.11-0.78] | 0.26 [0.07-0.60] | [0.02-0.13] |
| Correl. Coef. | 0.80 | 0.54 | 0.73 |

**Tableau 11 : Amplification des gènes ABL, B2M, GUS et CBFb-MYH11 dans la lignée cellulaire et chez des patients au diagnostic.**

| Echantillons | Nombre de Copie normalisé | | |
|---|---|---|---|
| | CBFb-MYH11/ ABL | CBFb-MYH11×100/ B2M | CBFb-MYH11/ GUS |
| Lignée cellulaire ME-1 | 0.90 | 4.64 | 0.40 |
| Patients | 1.35 | 3.98 | 0.42 |
| (n=24) | [0.13-14.8] | [1.45-447] | [0.07-4.57] |
| Correl. Coef. | 0.76 | 0.65 | 0.76 |

**Tableau 12 : Amplification des gènes ABL, B2M, GUS et AML1-ETO dans la lignée cellulaire et chez des patients au diagnostic.**

| Echantillons | Nombre de Copie Normalisé | | |
|---|---|---|---|
| | AML1-ETO /ABL | AML1-ETO x100/B2M | AML1-ETO /GUS |
| Lignée cellulaire KASUMI-1 | 5.46 | 80 | 1.42 |
| Patient | | | |

(suite)

| Echantillons | Nombre de Copie Normalisé | | |
|---|---|---|---|
| | AML1-ETO /ABL | AML1-ETO x100/B2M | AML1-ETO /GUS |
| Moelle | 1.5 | 5.2 | 0.43 |
| (n=8) | [0.26-3.0] | [0.26-11] | [0.054-1.5] |
| Sang | 2.1 | 7.8 | 0.48 |
| (n=13) | [0.17-5.9] | [0.21-170] | [0.027-2.7] |
| Correl. Coef. | 0.81 | 0.80 | 0.44 |

## REFERENCES BIBLIOGRAPHIQUES

[1] Marcucci et col., "Detection of minimal residual disease in patients with AML1/ETO-associated acute myeloid leukemia using a novel quantitative reverse transcription polymerase chain reaction assay", Leukemia, 1998, 12: 1482-1489.

[2] Van Dongen et col., Report of the European BIOMED 1 concerted Action, Leukemia, 1999.

[3] SAMBROOK et col., MOLECULAR CLONING a laboratory manual 2nd Ed., Cold Spring Harbor Laboratory Press, 1989.

## Revendications

1. Kit de dosage d'une séquence d'ADN d'intérêt à doser, **caractérisé en ce qu'**il comporte au moins une première gamme de dilutions de concentrations connues d'une construction comportant une séquence d'ADN correspondant à la séquence d'ADN d'intérêt à doser, insérée dans un vecteur et linéarisée et une seconde gamme de dilutions de concentrations connues d'une construction comportant une séquence d'un gène ou d'un ARN de gène ubiquitaire, insérée dans un vecteur et linéarisée, le milieu de dilution comportant en outre des acides nucléiques ne présentant pas d'homologie avec les séquences desdites constructions.

2. Kit selon la revendication 1, **caractérisée en ce que** la construction comportant une séquence d'ADN correspondant à la séquence d'ADN d'intérêt à doser, est entièrement artificielle.

3. Kit selon la revendication 1 ou 2, **caractérisée en ce que** le vecteur est un plasmide.

4. Kit selon l'une quelconque des revendications 1 à 3, **caractérisé en ce que** le milieu de dilution est une solution tampon.

5. Kit selon la revendication 4, **caractérisé en ce que** la solution tampon est de pH supérieur ou égal à 7.

6. Kit selon l'une quelconque des revendications 1 à 3, **caractérisé en ce que** l'acide nucléique ne présentant pas d'homologie avec les séquences d'intérêt est de l'ARN d'E. *coli.*

7. Kit selon l'une quelconque des revendications 1 à 6, **caractérisé en ce que** le gène ou l'ARN de gène ubiquitaire est choisi parmi le groupe composé d'ABL, GUS et B2M.

8. Kit selon l'une quelconque des revendications 1 à 7, **caractérisé en ce que** la séquence d'ADN d'intérêt à doser est un gène d'intérêt choisi parmi le groupe composé de AML1-ETO, E2A-PBX1, BCR-ABL, MLL-AF4, TEL-AML1, SIL-TAL, PML-RARA, CBFb-MYH11.

9. Kit selon l'une quelconque des revendications 1 à 8, **caractérisé en ce que** les gammes de dilutions sont conditionnées sous forme liquide ou lyophilisée.

**10.** Méthode de préparation d'un kit de dosage d'une séquence nucléotidique d'intérêt, **caractérisée en ce qu'**elle comporte les étapes suivantes:

- préparation d'une construction comprenant un vecteur, selon la méthode comportant les étapes suivantes :

(i) insertion d'une séquence d'ADN d'intérêt à doser dans le vecteur, et,
(ii) linéarisation de ladite construction par une enzyme de restriction sélectionnée de manière telle que ladite enzyme ne reconnaisse qu'un seul site de coupure sur le vecteur, à l'extérieur de l'insert,

- réalisation d'une gamme de dilutions de concentrations connues de ladite construction dans un milieu de pH supérieur ou égal à 7, comportant de l'ARN d'*E. coli*, et,
- réalisation d'une gamme de dilutions de concentrations connues d'une construction comportant une séquence d'un gène ou d'un ARN de gène ubiquitaire, insérée dans un vecteur et linéarisée.

**11.** Méthode selon la revendication 10, **caractérisé en ce que** la séquence d'ADN insérée dans le vecteur est choisie parmi le groupe composé de AML1-ETO, E2A-PBX1, BCR-ABL, MLL-AF4, TEL-AML1, SIL-TAL, PML-RARA, CBFb-MYH11.

**12.** Utilisation des kits selon l'une quelconque des revendications 1 à 9 pour la quantification d'acides nucléiques dans un échantillon à analyser.

**13.** Utilisation selon la revendication 12 pour la quantification de gènes.

**14.** Utilisation selon la revendication 12 pour la quantification de transcrits de fusion.

**15.** Utilisation selon la revendication 14 pour la quantification de transcrits de fusion impliqués dans les leucémies.

**16.** Méthode de quantification d'un acide nucléique cible dans un échantillon à analyser, **caractérisée en ce qu'**elle comporte les étapes suivantes :

- dosage par PCR quantitative et établissement d'une courbe d'étalonnage à l'aide de la gamme de dilutions d'une construction comportant une séquence d'ADN à doser, insérée dans un vecteur et linéarisée, ladite séquence d'ADN correspondant à la séquence nucléotidique cible,
- dosage par PCR quantitative et établissement d'une courbe d'étalonnage à l'aide de la gamme de dilutions d'une construction portant la séquence du gène ou ARN de gène ubiquitaire, insérée dans un vecteur et linéarisée,
- dosage par PCR quantitative de la séquence cible et du gène ou ARN de gène ubiquitaire dans l'échantillon, à l'aide des courbes d'étalonnage, et,
- calcul du nombre de copies normalisé à l'aide de la formule suivante :

$$\text{Log (NCN)} = \text{Log TF} - \text{Log GU}$$

Où NCN est le nombre de copies normalisé,
TF, le nombre de copies de la séquence cible, et
GU, le nombre de copies du gène ou ARN ubiquitaire, les dosages étant menés de préférence en double.

**17.** Méthode de diagnostic d'un patient atteint d'une leucémie impliquant un transcrit de fusion choisi parmi E2A-PBX1, MLL-AF4, TEL-AML1, BCR-ABL(mBCR), BCR-ABL(MBCR), SIL-TAL, PML-RARA, CBFb-MYH11, AML1-ETO comportant les étapes suivantes :

- mise en oeuvre d'un dosage dudit transcrit réalisé selon la méthode de quantification selon la revendication 16, et,
- comparaison du résultat avec une valeur de référence.

**18.** Utilisation de la méthode selon la revendication 17 pour apprécier l'efficacité d'un traitement anti-leucémique.

**Claims**

1. Kit for assaying a DNA sequence of interest to be assayed **characterized in that** it comprises at least a first dilution series of known concentrations of a construction comprising a DNA sequence corresponding to the DNA sequence of interest to be assayed, inserted into a vector and linearzied, and a second dilution series of known concentrations of a construction comprising a gene or RNA of an ubiquitous gene, inserted into a vector and linearized, the dilution medium moreover comprises nucleic acids having no homology with the sequences of interest of the calibrants.

2. The kit according to claim 1, wherein the construction comprising a DNA sequence corresponding to the DNA sequence of interest is totally artificial.

3. The kit according to claim 1 or 2, wherein the vector is a plasmid.

4. The kit according to anyone of claims 1 to 3, wherein the dilution medium is a buffer solution.

5. The kit according to claim 4, wherein the buffer solution has a pH greater than or equal to 7.

6. The kit according to anyone of claims 1 to 3, wherein the nucleic acid having no homology with the sequences of interest is a RNA of *E. coli.*

7. The kit according to any one of claims 1 to 6, wherein the gene or RNA of the ubiquitous gene is chosen from the group composed of ABL, GUS and B2M.

8. The kit according to any one of claims 1 to 7, wherein the sequence of the gene of interest to be assayed is a gene of interest eslected from the group composed of AML1-ETO, E2A-PBX1, BCR-ABL, MLL-AF4, TEL-AML1, SIL-TAL, PML-RARA, CBFb-MYH11.

9. The kit according to any one of claims 1 to 8, wherein the dilution series are packaged in liquid or lyophilized form.

10. A method for preparing a kit for assaying a nucleotide sequence of interest, comprising the following steps:

    - preparing a construction comprising a vector according to the method comprising the following steps of

        (i) inserting the DNA sequence of interest to be assayed into the vector, and
        (ii)linearizing said construction by a restriction enzyme selected so that said enzyme recognizes only one breakdown site on the vector, outside of the insert,

    - producing a dilution series of known concentrations of said a construction in a medium with a pH greater than or equal to 7, comprising RNA of *E. coli.,* and
    - producing a dilution series of known concentrations of construction comprising a sequence of a gene or RNA of ubiquitous gene, inserted into the vector and linearized.

11. The method of claim 10, wherein the DNA sequence inserted into the vector is selected from the group composed of AML1-ETO, E2A-PBX1, BCR-ABL, MLL-AF4, TEL-AML1, SIL-TAL, PML-RARA, CBFb-MYH11.

12. Use of the kits according to anyone of claims 1 to 9, for the quantification of nucleic acids in a sample to be assayed.

13. The use according to claim 12, for the quantification of genes.

14. The use according to claim 12, for the quantification of fusion transcripts.

15. The use according to claim 14, for the quantification of fusion transcripts involved in leukaemia.

16. A method for the quantification of a target nucleic acid in a sample to be analysed, comprising the following steps:

    - assay by quantitative PCR and establishment of a calibration curve using the dilution series of a construction comprising a DNA sequence to be assayed, inserted into a vector and linearized, said DNA sequence corresponding to the target nucleotidic sequence,

- assay by quantitative PCR and establishment of a calibration curve using the dilution series of a construction carrying the sequence of the gene or RNA of ubiquitous gene,
- assay by quantitative PCR of the target sequence and of the gene or RNA of ubiquitous gene in the sample, using calibration curves, and,
- calculation of the standardized number of copies using the following formula:

$$\texttt{Log (SNC) = Log TF - Log UG}$$

where SNC is the standardized number of copies,
TF the number of copies of the target sequence, and
UG the number of copies of the ubiquitous gene or RNA, the assays preferably being duplicated.

17. A method of diagnostic of a patient having a leukemia involving a fusion transcript chosen amongst E2A-PBX1, MLL-AF4, TEL-AML1, BCR-ABL (MBCR), BCR-ABL (MBCR), SIL-TAL, PML-RARA, CBFb-MYH11, AML1-ETO, comprising the following steps:

- carrying out an assay of said transcript performed according to the quantification method according to claim 16, and
- comprising the result to a reference value.

18. Use of the method according to claim 17, to appreciate the efficiency of an anti-leukemic treatment.

**Patentansprüche**

1. Kit zur Bestimmung einer zu bestimmenden interessierenden DNA-Sequenz, **dadurch gekennzeichnet, dass** er wenigstens Folgendes umfasst: eine erste Verdünnungsreihe von bekannten Konzentrationen einer Konstruktion, die eine DNA-Sequenz, die der zu bestimmenden interessierenden DNA-Sequenz entspricht, umfasst und die in einen Vektor eingefügt und linearisiert ist, und eine zweite Verdünnungsreihe von bekannten Konzentrationen einer Konstruktion, die eine Sequenz eines ubiquitären Gens oder einer RNA eines ubiquitären Gens umfasst und die in einen Vektor eingefügt und linearisiert ist, wobei das Verdünnungsmedium weiterhin Nucleinsäuren umfasst, die keine Homologie mit den Sequenzen der Konstruktionen aufweisen.

2. Kit gemäß Anspruch 1, **dadurch gekennzeichnet, dass** die Konstruktion, die eine DNA-Sequenz, die der zu bestimmenden interessierenden DNA-Sequenz entspricht, umfasst, vollständig künstlich ist.

3. Kit gemäß Anspruch 1 oder 2, **dadurch gekennzeichnet, dass** der Vektor ein Plasmid ist.

4. Kit gemäß einem der Ansprüche 1 bis 3, **dadurch gekennzeichnet, dass** das Verdünnungsmedium eine Pufferlösung ist.

5. Kit gemäß Anspruch 4, **dadurch gekennzeichnet, dass** die Pufferlösung einen pH-Wert von größer oder gleich 7 aufweist.

6. Kit gemäß einem der Ansprüche 1 bis 3, **dadurch gekennzeichnet, dass** es sich bei der Nucleinsäure, die keine Homologie mit den interessierenden Sequenzen aufweist, um RNA von *E. coli* handelt.

7. Kit gemäß einem der Ansprüche 1 bis 6, **dadurch gekennzeichnet, dass** das ubiquitäre Gen oder die RNA des ubiquitären Gens aus der Gruppe ausgewählt ist, die aus ABL, GUS und B2M besteht.

8. Kit gemäß einem der Ansprüche 1 bis 7, **dadurch gekennzeichnet, dass** die zu bestimmende interessierende DNA-Sequenz ein interessierendes Gen ist, das aus der Gruppe ausgewählt ist, die aus AML1-ETO, E2A-PBX1, BCR-ABL, MLL-AF4, TEL-AML1, SIL-TAL, PML-RARA, CBFb-MYH11 besteht.

9. Kit gemäß einem der Ansprüche 1 bis 8, **dadurch gekennzeichnet, dass** die Verdünnungsreihen in flüssiger oder lyophilisierter Form konditioniert sind.

**10.** Verfahren zur Herstellung eines Kits zur Bestimmung einer interessierenden Nucleotidsequenz, **dadurch gekennzeichnet, dass** es die folgenden Schritte umfasst:

- Herstellung einer Konstruktion, die einen Vektor umfasst, nach einem Verfahren, das die folgenden Schritte umfasst:

(i) Einfügen einer zu bestimmenden interessierenden DNA-Sequenz in den Vektor; und
(ii) Linearisieren der Konstruktion mit einem ausgewählten Restriktionsenzym, so dass das Enzym nur eine einzige Spaltstelle auf dem Vektor außerhalb der Einfügung erkennt;

- Herstellen einer Verdünnungsreihe von bekannten Konzentrationen der Konstruktion in einem Medium mit einem pH-Wert von größer oder gleich 7, das RNA von *E. coli* umfasst; und
- Herstellen einer Verdünnungsreihe von bekannten Konzentrationen einer Konstruktion, die eine Sequenz eines ubiquitären Gens oder einer RNA eines ubiquitären Gens umfasst und die in einen Vektor eingefügt und linearisiert ist.

**11.** Verfahren gemäß Anspruch 10, **dadurch gekennzeichnet, dass** die in den Vektor eingefügte DNA-Sequenz aus der Gruppe ausgewählt ist, die aus AML1-ETO, E2A-PBX1, BCR-ABL, MLL-AF4, TEL-AML1, SIL-TAL, PML-RARA, CBFb-MYH11 besteht.

**12.** Verwendung der Kits gemäß einem der Ansprüche 1 bis 9 zur Quantifizierung von Nucleinsäuren in einer zu analysierenden Probe.

**13.** Verwendung gemäß Anspruch 12 zur Quantifizierung von Genen.

**14.** Verwendung gemäß Anspruch 12 zur Quantifizierung von Fusionstranscripten.

**15.** Verwendung gemäß Anspruch 14 zur Quantifizierung von Fusionstranscripten, die an Leukämien beteiligt sind.

**16.** Verfahren zur Quantifizierung einer Zielnucleinsäure in einer zu analysierenden Probe, **dadurch gekennzeichnet, dass** es die folgenden Schritte umfasst:

- Bestimmung durch quantitative PCR und Herstellen einer Eichkurve mit Hilfe der Verdünnungsreihe einer Konstruktion, die eine zu bestimmende DNA-Sequenz umfasst und die in einen Vektor eingefügt und linearisiert ist, wobei die DNA-Sequenz der Zielnucleotidsequenz entspricht;
- Bestimmung durch quantitative PCR und Herstellen einer Eichkurve mit Hilfe der Verdünnungsreihe einer Konstruktion, die die Sequenz des ubiquitären Gens oder der RNA des ubiquitären Gens umfasst und die in einen Vektor eingefügt und linearisiert ist;
- Bestimmung der Zielsequenz und des ubiquitären Gens oder der RNA des ubiquitären Gens durch quantitative PCR in der Probe mit Hilfe der Eichkurven; und
- Berechnung der normierten Zahl der Kopien mit Hilfe der folgenden Formel:

$$\log (NCN) = \log TF - \log GU,$$

wobei NCN die normierte Zahl der Kopien ist;
TF die Zahl der Kopien der Zielsequenz ist; und
GU die Zahl der Kopien des ubiquitären Gens oder der ubiquitären RNA ist, wobei die Bestimmungen vorzugsweise doppelt durchgeführt werden.

**17.** Diagnoseverfahren für einen Patienten, der unter einer Leukämie leidet, die ein Fusionstranscript impliziert, das aus E2A-PBX1, MLL-AF4, TEL-AML1, BCR-ABL(mBCR), BCR-ABL(MBCR), SIL-TAL, PML-RARA, CBFb-MYH11, AML1-ETO ausgewählt ist, umfassend die folgenden Schritte:

- Durchführen einer Bestimmung des Transcripts nach dem Verfahren zur Quantifizierung gemäß Anspruch 16; und
- Vergleich des Ergebnisses mit einem Referenzwert.

**18.** Verwendung des Verfahrens gemäß Anspruch 17 zur Bewertung der Wirksamkeit einer Leukämiebehandlung.

Figure 1

Niveaux d'expression des transcrits de fusion
- patients leucémiques au diagnostic -

Transcrits de fusion

*mBA : mBCR-ABL
MBA : MBCR-ABL

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **MARCUCCI.** Detection of minimal residual disease in patients with AML1/ETO-associated acute myeloid leukemia using a novel quantitative reverse transcription polymerase chain reaction assay. *Leukemia,* 1998, vol. 12, 1482-1489

- **VAN DONGEN.** Report of the European BIOMED 1 concerted Action. *Leukemia,* 1999
- **SAMBROOK.** MOLECULAR CLONING. Cold Spring Harbor Laboratory Press, 1989